# EUROPEAN PATENT APPLICATION

(11) **EP 4 847 083 A1**
(43) Date of publication of application: **02.12.2026**
(21) Application number: 26178738.6
(22) Date of filing: 14.05.2026
(51) Int. Cl.: A61N 5/06

(54) **LED LIGHTING SYSTEM FOR OPTOGENETIC APPLICATIONS**

(30) Priority: 27.05.2025 IT 202500012142
(71) Applicant: CyNexo S.r.l., 33050 Trivignano Udinese (UD) (IT)
(72) Inventor: PARUSSO, Stefano, 33030 Campoformido (UD) (IT); MOIMAS, Diego, 33050 San Vito Al Torre (UD) (IT); DELPIN, Daniele, 34070 Savogna D'Isonzo (GO) (IT)
(74) Representative: Frare, Paolo

(57) **Abstract**

The present finding relates to a device (1) for light stimulation, particularly for use in optogenetics, comprising: a support base (2); an LED (6), supported by the support base (2); an electronic board (8), supported by the support base (2) and configured to control the operation of the LED (6); a first light radiation conveying group (12) comprising first light radiation conveying means (13) removably associated with the support base (2) by means of removable fixing means (14), in such a way as to face said LED (6) and to convey the light radiation emitted thereby towards the outside of the device (1), wherein the first conveying means (13) are removable from the support base (2) by acting on the removable fixing means (14), in such a way as to allow replacement of the first conveying means (13) with second light radiation conveying means (15) configured to convey the light radiation emitted by the LED (6) towards the outside of the device (1) in a different way with respect to the first conveying means (13). The finding also relates to a method of reconfiguring such device and to a light radiation conveying kit, particularly for use in optogenetics.

## Description

The present finding relates to an LED lighting system for optogenetic applications, and in particular to a device for light stimulation, particularly for use in optogenetics, to a method of reconfiguring such device, and to a light radiation conveying kit, particularly for use in optogenetics.

Nowadays, devices for light stimulation using LEDs for use in optogenetics are known, provided with systems for conveying, towards the outside of the device, the light produced by an LED present inside the device itself.

Depending on the specific use for which they are intended (in vitro or in vivo), such prior-art devices exhibit a specific and distinct configuration and structure.

However, such prior-art solutions exhibit certain limitations; indeed, prior-art devices designed for in vivo use generally cannot be used for in vitro applications, and vice versa, typically due to the specific configuration of their conveying systems, which are optimized for such specific applications.

A user who needs to perform both in vivo and in vitro treatments is therefore forced to obtain both one or more light stimulation devices adapted to in vivo applications and one or more devices for light stimulation adapted to in vitro applications, which entails the need to have adequate storage space for storing both typologies of devices.

Furthermore, the need to have available at least one device for light stimulation adapted to in vivo applications and at least one device for light stimulation adapted to in vitro applications results in relatively high costs for users who need to perform both in vivo and in vitro treatments.

The main task of the present finding is to overcome the above-mentioned drawbacks, and in particular to allow to equip oneself for carrying out light stimulations in the optogenetic field both in vivo and in vitro, while reducing the overall storage space required for the equipment necessary for such stimulations with respect to the cited prior art.

Within such task, an object of the finding is to reduce the overall cost of the equipment required for carrying out light stimulations in the optogenetic field both in vivo and in vitro.

The above object and task are achieved by a device for light stimulation particularly for use in optogenetics, comprising:
- a support base;
- an LED, supported by the support base;
- an electronic board, supported by the support base and configured to control the operation of the LED;
- a first light radiation conveying group comprising first light radiation conveying means associated with the support base in a removable manner, by means of removable fixing means, in such a way as to face the LED and to convey the light radiation emitted thereby towards the outside of the device,
wherein the first conveying means are removable from the support base by acting on the removable fixing means, in such a way as to allow replacement of the first conveying means with second light radiation conveying means configured to convey the light radiation emitted by the LED towards the outside of the device in a different way with respect to the first conveying means.

It should be noted that, in the present text, the expression "to convey the light radiation emitted by the LED towards the outside of the device in a different way with respect to the first conveying means" indicates that the second conveying means convey the light radiation towards the outside of the device using a technology that is at least partially different from that used by the first conveying means; for example, the first conveying means may use an optical fibre technology, whereas the second conveying means may use a lens and/or an optical diffuser, or vice versa, and so on.

It should be noted that, in the present text, stating that two components are fixed together in a "removable" manner means that such components are fixed together in such a way that they can be separated and, possibly, fixed together again, without requiring the breaking of such components or of any connection means therebetween.

It should be noted that, in the present text, stating that two components are fixed together in a "non-removable" manner means that such components are fixed together in such a way that they cannot be separated from one another unless one or both of such components and/or any connection means therebetween are broken or damaged.

In an advantageous embodiment, the first conveying group comprises a first support structure, fixed to the support base, by which the first conveying means are supported.

In an advantageous embodiment, the first support structure is fixed (removably or non-removably) to the support base, and the first conveying means and the second conveying means can be fixed, alternatively to one another, in a removable manner, by means of the removable fixing means, to the support structure.

In a further advantageous embodiment, the first support structure is fixed in a removable manner, by means of the removable fixing means, to the support base.

In an advantageous embodiment, the first conveying means can be associated with the support base, and removed therefrom, together with the first support structure.

In an advantageous embodiment, the second conveying means form part of a second conveying group comprising a second support structure, fixed to the support base, by which the second conveying means are supported.

In an advantageous embodiment, the second conveying means can be associated with the support base, and removed therefrom, together with the second support structure.

In an advantageous embodiment, the first light radiation conveying means comprise:
- a support element, removably associated with the support base by means of the removable fixing means;
- a connection structure, supported by the support element, configured to stably support a first optical fibre in such a way that a first free end of such first optical fibre faces, or can be arranged to face, the LED, and configured to accommodate and fix, in a removable manner, a second optical fibre in such a way that a free end of such second optical fibre faces a second free end of the first optical fibre.

In an advantageous embodiment, the first conveying means are configured to allow translation of the first free end of the first optical fibre along a first direction perpendicular to the plane of arrangement of the LED, so as to be able to bring it closer to, or further away from, the LED.

In an advantageous embodiment, the first conveying means are configured to allow movement of the first free end of the first optical fibre along a first plane parallel to the plane of arrangement of the LED.

In an advantageous embodiment, the second light radiation conveying means comprise:
- a lens, or a tubular structure comprising a series of lenses in series with each other, configured to be positioned with a first face thereof facing the LED and to convey the light radiation emitted by the LED along a given direction;
- a screen, transparent or partially transparent, facing a second face of the lens or tubular structure, placed on the opposite side to the first face.

The above object and task are also achieved by a method of reconfiguring a device according to the finding.

The method comprises the following steps:
a) arranging a device according to the finding comprising a first light radiation conveying group;
b) removing the first conveying means from the support base of the device by acting on the removable fixing means;
c) fixing to the support base of the device, by acting on the removable fixing means, second light radiation conveying means configured to convey the light radiation emitted by the LED towards the outside of the device in a different way with respect to the first conveying means.

In an advantageous embodiment, step b) comprises the step of removing the first conveying means from the first support structure by acting on the removable fixing means.

In an advantageous embodiment, step c) comprises the step of fixing the second conveying means to the first support structure by acting on the removable fixing means.

In an advantageous embodiment, step b) comprises the step of removing the first conveying means from the support base, together with the first support structure, by acting on the removable fixing means.

In an advantageous embodiment, step c) comprises the step of fixing the second conveying means to the support base, together with the second support structure, by acting on the removable fixing means.

The above object and task are also achieved by a light radiation conveying kit.

The conveying kit according to the finding comprises:
- a support base;
- an LED, supported by the support base;
- an electronic board, supported by the support base and configured to control the operation of the LED;
- a first light radiation conveying group comprising first light radiation conveying means that can be removably associated with the support base, by means of removable fixing means, so as to be positionable facing the LED and to convey the light radiation emitted thereby towards the outside of the device, wherein the first conveying means are removable from the support base by acting on the removable fixing means;
- a second light radiation conveying group comprising second light radiation conveying means that can be removably associated with the support base, by means of removable fixing means, so as to be positionable facing the LED and to convey the light radiation emitted thereby towards the outside of the device in a different way with respect to the first conveying means.

The features and advantages of the present finding will become more apparent from the following exemplary and non-limiting description, with reference to the attached schematic drawings, in which:

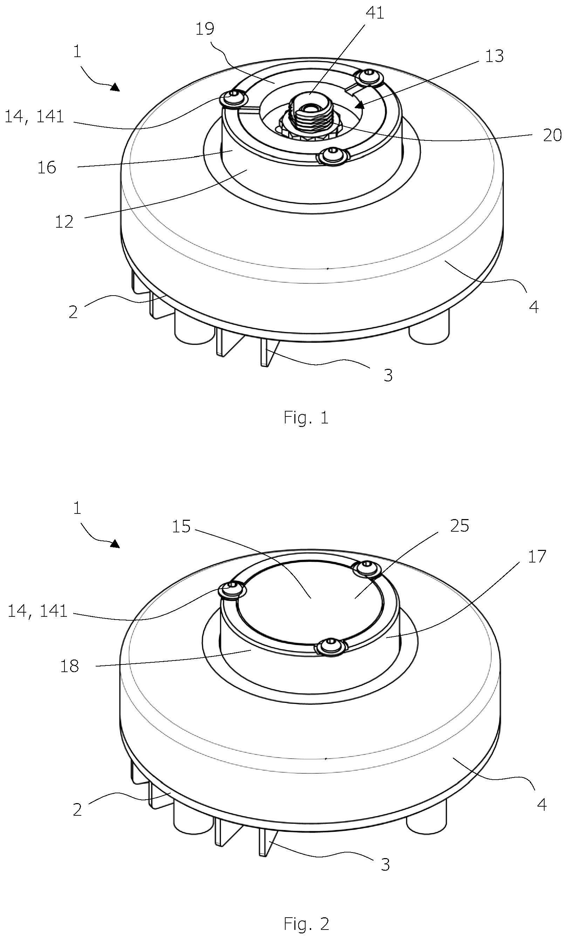

- Figure 1 is a perspective view of a device for light stimulation according to the finding, configured for use in in vivo applications;
- Figure 2 is a perspective view of a device for light stimulation according to the finding, configured for use in in vitro applications;

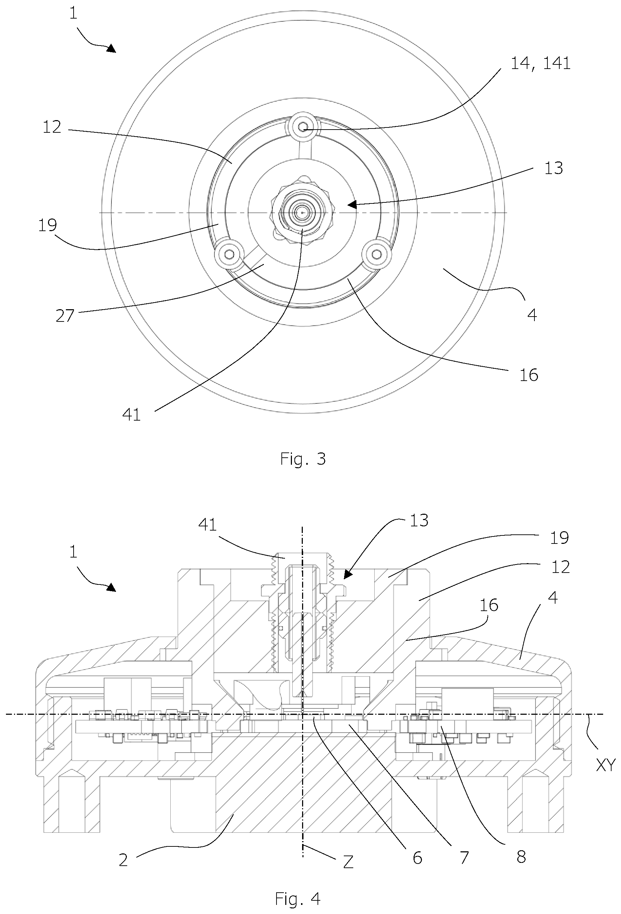

- Figure 3 is a top view of the device for light stimulation of Figure 1;
- Figure 4 is a lateral sectional view of the device for light stimulation of Figure 1;

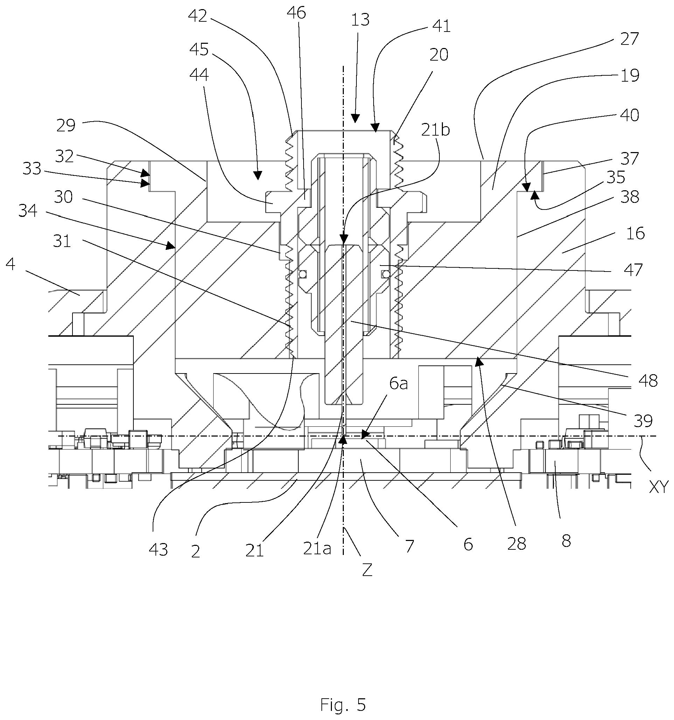

- Figure 5 is an enlarged detail of Figure 4;

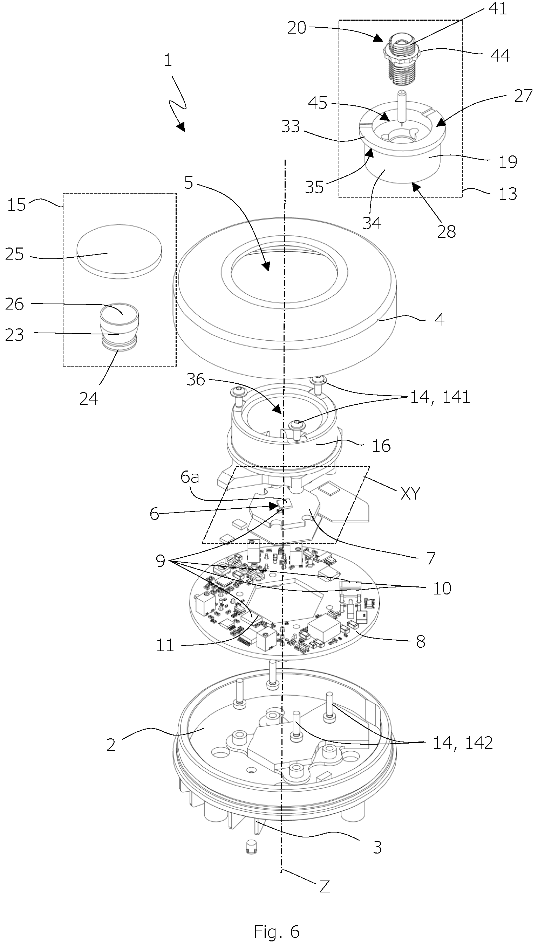

- Figure 6 is an exploded view of a light radiation conveying kit according to the finding.

With reference to the attached figures, number 1 generally indicates a device according to the finding used for light stimulation in optogenetic applications that may be performed in vitro or in vivo.

The device 1 according to the finding comprises a support base 2.

Advantageously, the support base 2 has a preferably box-shaped configuration, more preferably with a circular plan shape.

Advantageously, the support base 2 comprises heat dissipation means.

Advantageously, the support base 2 is made of aluminium or copper, and the heat dissipation means preferably comprise fins 3, preferably made of aluminium or copper, preferably projecting from the bottom of the support base 2, more preferably formed in one piece with the support base 2, suitable for promoting heat dissipation.

Advantageously, the support base 2 may be a standalone element, as in the advantageous examples of the attached figures, or it may be integrated into a more complex system, such as, for example, an optical bench, not illustrated.

Advantageously, the device 1 according to the finding comprises a cover 4, preferably having a box-shaped configuration, more preferably with a circular plan shape.

Advantageously, the cover 4 is made of metal, preferably copper, aluminium, or steel; in other advantageous embodiments, the cover 4 may be made of plastic, of the thermally conductive or non-thermally conductive type.

Advantageously, the cover 4 comprises an opening 5, preferably having a circular section, preferably formed centrally in the cover 4.

Advantageously, the cover 4 is fixed, preferably by screwing or snap-fitting, to the support base 2, thereby forming therewith an outer shell of the device 1, whose inside communicates with the outside of the device 1 through the opening 5 of the cover 4.

The device 1 according to the finding comprises an LED 6, supported by the support base 2.

Advantageously, the LED 6 is fixed to an electronic sub-board 7, advantageously configured to power and/or control the same, preferably made of a flat substrate of metallic material, for example copper or aluminium (this type of electronic sub-board is generally referred to as a "Metal Core Printed Circuit Board" (MCPCB)), so as to dissipate the heat generated by the LED 6 during use. Advantageously, the LED 6 comprises a flat face 6a, from which light is emitted, advantageously parallel to the electronic sub-board 7.

Advantageously, the electronic sub-board 7 is in direct or indirect contact (for example with the interposition of further sheets or layers made of materials having good thermal conductivity, for example copper or aluminium, and/or with the interposition of a thermally conductive paste) with the support base 2, in such a way that the heat generated by the LED 6 during its operation is transferred to the support base 2 and can then be dissipated to the outside by the latter through its heat dissipation means (for example the fins 3).

The device 1 further comprises an electronic board 8 supported by the support base 2 and configured to control the operation of the LED 6.

Advantageously, the electronic board 8 is operatively connected to the electronic sub-board 7, in such a way as to control the latter and, consequently, the LED 6; alternatively, the electronic sub-board 7 may be integrated into the electronic board 8, thereby constituting a part of it.

Advantageously, the electronic board 8 may be connected to the electrical mains of a building and/or to an electrical power generator (for example a battery, outside or inside the device 1), for example by means of a connector, not illustrated, preferably of the coaxial type, and an electrical cable, not illustrated.

Advantageously, the electronic board 8 may be operatively connected to control means, not illustrated, external to the device 1, for example by means of a connector, preferably of the coaxial type, and an electrical cable, not illustrated; such control means may comprise, for example, a computer, a PLC, etc.

In advantageous embodiments, such as the one illustrated in the attached figures, the device 1 comprises detection means 9, configured to perform detections of one or more parameters relating to the operation of the LED 6, and advantageously operatively controlled by the electronic board 8.

Advantageously, such one or more parameters relating to the operation of the LED 6 may comprise the electric current absorbed by the LED 6; in such a case, the detection means 9 preferably comprise one or more electrical resistors 10 and a connector, not illustrated in the attached figures, preferably of the coaxial type, connected or connectable, for example by means of a dedicated electrical cable, not illustrated, to current-value reading means, not illustrated in the attached figures, external to the device 1, such as a computer.

Advantageously, the one or more parameters relating to the operation of the LED 6 detected by the detection means 9 may comprise the intensity of the light radiation emitted by the LED 6; in such a case, the detection means 9 preferably comprise one or more optical sensors 11 and a connector, not illustrated in the attached figures, preferably of the coaxial type, connected or connectable, for example by means of a dedicated electrical cable, not illustrated, to means for reading and/or processing a value dependent on the intensity of the light radiation emitted by the LED 6, not illustrated, external to the device 1, such as for example a computer.

The device 1 comprises a first light radiation conveying group 12 comprising first light radiation conveying means 13 removably associated with the support base 2 by means of removable fixing means 14, in such a way as to face said LED 6 and to convey the light radiation emitted thereby towards the outside of the device 1.

Advantageously, the first conveying group 12 partially projects outside the cover 4 through the opening 5.

The first conveying means 13 are removable from the support base 2 by acting on the removable fixing means 14, in such a way as to allow replacement of the first conveying means 13 with second light radiation conveying means 15 configured to convey the light radiation emitted by the LED 6 towards the outside of the device 1 in a different way with respect to the first conveying means 13.

In advantageous embodiments, the first conveying group 12 comprises a first support structure 16 fixed to the support base 2, by which the first conveying means 13 are supported.

In advantageous embodiments, such as the one illustrated in the attached figures, the first support structure 16 is preferably resting on the electronic board 8.

Advantageously, the first support structure 16 is made of aluminium, preferably anodized aluminium.

Advantageously, the first support structure 16 is substantially shaped as a cylinder or a prism.

Advantageously, the first support structure 16 comprises a first through hole 36, preferably comprising, starting from its end facing the opposite side to the support base 2, a first portion 37 and a second portion 38, contiguous to each other and preferably cylindrical, and a third portion 39, preferably tapered. The diameter of the first portion 37 is advantageously greater than the diameter of the second portion 38, thereby defining, at the connection zone between the two portions, a first abutement 40, advantageously annular.

In advantageous embodiments, the first support structure 16 is fixed to the support base 2, and the first conveying means 13 and the second conveying means 15 can be fixed, alternatively to one another, in a removable manner, by means of the removable fixing means 14, to the first support structure 16; in such advantageous embodiments, the first support structure 16 may advantageously be fixed to the support base 2 either in a non-removable manner or in a removable manner.

In such advantageous embodiments, the removable fixing means 14 comprise first fixing screws 141 by means of which the first conveying means 13 and the second conveying means 15 can be fixed, alternatively to one another, in a removable manner, to the first support structure 16, which may advantageously be fixed to the support base 2 either in a non-removable manner or in a removable manner.

In advantageous embodiments, the first support structure 16 is fixed in a removable manner to the support base 2 by means of the removable fixing means 14.

In advantageous embodiments, the removable fixing means 14 comprise second fixing screws 142 by means of which the first support structure 16 is fixed, in a removable manner, to the support base 2.

In advantageous embodiments, in which the first support structure 16 is fixed in a removable manner to the support base 2, the first conveying means 13 may advantageously be associated with the support base 2 and removed therefrom together with the first support structure 16.

In advantageous embodiments, in which the first support structure 16 is fixed in a removable manner to the support base 2, the second conveying means 15 may advantageously form part of a second conveying group 17 comprising a second support structure 18 fixed or fixable to the support base 2, by which the second conveying means 15 are supported; in such advantageous embodiments, the second conveying means 15 may advantageously be associated with the support base 2 and removed therefrom together with the second support structure 18.

In advantageous embodiments, the first light radiation conveying means 13 comprise a support element 19 removably associated with the support base 2, preferably through the first support structure 16, which is in turn fixed to the support base 2 by means of the removable fixing means 14.

Advantageously, the support element 19 is made of aluminium, preferably anodized aluminium.

Advantageously, the support element 19 has an external shape that is at least partially counter-shaped to the first hole 36, so that it can be inserted therein.

Advantageously, the support element 19 has an external shape such that, once inserted into the first hole 36, it can be rotated about the longitudinal axis of the first hole 36 (coinciding with its own longitudinal axis); advantageously, rotation of the support element 19 within the first hole 36 can be locked by means of the removable fixing means 14.

Advantageously, the angular position of the support element 19 inside the first hole 36 may be adjusted with the aid of reference means such as, for example, a dedicated first notch, not illustrated, formed on the first support structure 16 at a position corresponding to the outer edge of the first hole 36, and a second notch, not illustrated, formed on a first end 27 of the support element 19, which advantageously lies flush with the outer edge of the first hole 36, in such a way that the correct angular position of the support element 19 with respect to the first hole 36 can be obtained by aligning the first and second notches with each other.

Preferably, in the advantageous case in which the first hole 36 comprises the first portion 37, the second portion 38, and the third portion 39 described above, the support element 19 comprises an outer surface 32 which, proceeding from the first end 27 to the second end 28 of the support element 19, comprises a first section 33, preferably cylindrical, advantageously counter-shaped to the inner surface of the first portion 37 of the first hole 36, and a second section 34, preferably cylindrical, having a diameter smaller than that of the first section 33 and advantageously counter-shaped to the inner surface of the second portion 38 of the first hole 36.

Advantageously, the outer surface 32 of the support element 19 comprises a second abutement 35, annular in shape, connecting the first section 33 and the second section 34.

Advantageously, the support element 19 can be inserted into the first hole 36 of the first support structure 16 with its second end 28 facing the support base 2 and with the second abutement 35 resting against the first abutement 40 of the first hole 36.

Advantageously, the support element 19 is traversed by a second through hole 45, at least partially threaded, which, preferably, proceeding from the first end 27 to the second end 28 of the support element 19, comprises a first section 29, preferably cylindrical, a second section 30, preferably cylindrical, having a diameter smaller than that of the first section 29, and a third section 31, preferably cylindrical and threaded, preferably having a diameter smaller than that of the second section 30.

Advantageously, the first conveying means 13 comprise a connection structure 20, supported by the support element 19, configured to stably support a first optical fibre 21 in such a way that a first free end 21a of the first optical fibre 21 faces, or can be arranged to face, the LED 6; advantageously, the connection structure 20 is configured to accommodate and fix therein, in a removable manner, a second optical fibre, not illustrated, in such a way that a free end of the second optical fibre faces a second free end 21b of the first optical fibre 21, so as to be able to exchange a light signal therewith.

In advantageous embodiments, such as for example the one illustrated in the attached figures, the first conveying means 13 are configured to allow translation of the first free end 21a of the first optical fibre 21 along a first direction "Z" perpendicular to the plane of arrangement of the LED 6, so as to be able to bring it closer to, or further away from, the LED 6 itself.

It should be noted that the plane of arrangement of the LED 6 means an ideal plane to which the LED 6 is fixed; in the advantageous embodiment illustrated in the attached figures, such plane of arrangement corresponds to the preferably flat face of the electronic sub-board 7 to which the LED 6 is fixed; advantageously, such plane of arrangement is parallel to the flat face 6a of the LED 6 from which the latter emits light radiation.

Advantageously, the direction Z is parallel to the longitudinal axis of the second hole 45.

In advantageous embodiments, the connection structure 20 advantageously comprises a connector 41 inserted into the second hole 45, having a tubular configuration, whose outer surface is partially counter-shaped to the inner surface of the second hole 45 and at least partially counter-threaded to the threaded region of such second hole 45, so that such connection structure 20 can advance or retract within the second hole 45, along the direction of its longitudinal axis, by screwing.

Advantageously, the connection structure 20 comprises a first end 42, preferably externally threaded, and an opposite second end 43, whose outer surface is advantageously counter-shaped to the third section 31 of the second hole 45 and is counter-threaded to such third section 31, so as to be able to advance or retract within such third section 31 by screwing.

Advantageously, the connection structure 20 comprises an annular projection 46 radially protruding from the inner surface of the connector 41. The annular projection 46 is preferably positioned between the first end 42 and the second end 43 of the connector 41.

Advantageously, an annular ring nut 44 protrudes from the outer lateral surface of the connector 41, said annular ring nut being suitable for facilitating screwing thereof within the second hole 45 and being positioned between the first end 42 and the second end 43 of the connector 41 itself.

In advantageous embodiments, the connection structure 20 comprises a sleeve element 47 stably fixed, for example by geometric interference or by other fixing means of a known type, such as for example gluing, welding, etc., inside the connector 41, advantageously between the annular projection 46 and the second end 43 of the connector 41.

In advantageous embodiments, the connection structure 20 comprises a ferrule 48 having a tubular configuration, inserted into the sleeve element 47, advantageously under conditions of geometric interference. Advantageously, the condition of geometric interference ensures that the ferrule 48 cannot move within the sleeve element 47 and therefore relative to the connector 41.

Alternatively, the ferrule 48 may be fixed inside the sleeve element 47 by other fixing means of a known type, such as for example gluing, welding, etc.

Advantageously, the second free end 21b of the first optical fibre 21 is fixed within the ferrule 48 in such a way as to be accessible from the outside thereof, so as to be able to transmit a light signal outside the ferrule 48.

Advantageously, the second end 43 of the connector 41 is screwed to the support element 19 in such a way that the first free end 21a of the first optical fibre 21 faces the LED 6.

Advantageously, by rotating the connector 41 clockwise or counterclockwise with respect to the support element 19, it is possible to move the first free end 21a of the first optical fibre 21 and the LED 6 farther apart or closer together, respectively, by screwing or unscrewing the second end 43 of the connection structure 20 within the third section 31 of the second hole 45.

In advantageous embodiments, such as for example the one illustrated in the attached figures, the first conveying means 13 are configured to allow movement of the first free end 21a of the first optical fibre 21 along a first XY plane parallel to the plane of arrangement of the LED 6; it should be noted that the first XY plane is parallel to the plane of arrangement of the LED 6 and advantageously parallel to the flat face 6a of the LED 6.

In advantageous embodiments, movement of the first free end 21a of the first optical fibre 21 along the first XY plane is achieved by forming the second hole 45, in which the connection structure 20 is positioned, in a slightly off-centre position with respect to the support element 19, so that, by rotating the support element 19 within the first hole 36, the longitudinal axis of such support element 19, and consequently the first free end 21a of the first optical fibre 21, which is positioned on such axis, describes an ellipse with respect to the first XY plane.

The possibility of performing such movement is particularly advantageous, since it allows the first free end 21a to be brought at a desired region of the LED 6 and then to fix the first free end 21a in such position, for example by tightening the first fixing screws 141; in this way, it is advantageously possible to position the first free end 21a facing a different region of the flat face 6a of the LED 6, for example if a region thereof previously faced by such free end 21a exhibits defects and/or reduced performance.

In advantageous embodiments, the second light radiation conveying means 15 comprise a lens 23, or a tubular structure comprising a series of lenses in series with each other, configured to be positioned with a first face 24 thereof facing the LED 6 and to convey the light radiation emitted by the LED 6 along a given direction.

In such advantageous embodiments, the second light radiation conveying means 15 comprise a screen 25, transparent or partially transparent, facing a second face 26 placed on the opposite side to the first face 24.

Advantageously, the screen 25 may be fixed, preferably but not necessarily in a non-removable manner, for example by gluing, to the second face 26, either directly or with the interposition of further optical elements, not illustrated, interposed between such screen 25 and the second face 26 and constrained, preferably but not necessarily in a non-removable manner, to such components.

In other advantageous embodiments, not illustrated, the first conveying means 13 or the second conveying means 15 may have different configurations and shapes.

For example, the first conveying means 13 or the second conveying means 15 may comprise a Fresnel lens, or telecentric optics, or camera optics.

For example, in advantageous embodiments, not illustrated, the first conveying means 13 may comprise the connection structure 20 but, instead of the support element 19, an optical assembly (for example of the type typically used for video surveillance) having a cylindrical stem externally counter-threaded to the third section 31 of the second hole 45, so as to be insertable by screwing to a desired depth inside such third section 31.

The present finding also relates to a method of reconfiguring a device 1 according to the finding.

The method according to the finding comprises a step a), in which a device 1 is arranged, comprising a first light radiation conveying group 12.

The method according to the finding comprises a step b), in which the first conveying means 13 are removed from the support base 2 of the device 1 by acting on the removable fixing means 14.

In a first advantageous embodiment, during step b), the first fixing screws 141 are removed by unscrewing them from the first support structure 16. Once the first fixing screws 141 have been removed, the support element 19 is removed by extracting it from the first support structure 16, thereby also removing the connection structure 20.

In a second advantageous embodiment, in which the first support structure 16 is fixed in a removable manner to the support base 2, during step b) it is advantageously possible, by acting on the removable fixing means 14 (for example by removing the second fixing screws 142, if present), to separate the first support structure 16, and the first conveying means 13 supported thereby, from the support base 2.

The method according to the finding further comprises a step c), in which second light radiation conveying means 15 are fixed to the support base 2 of the device 1, by acting on the removable fixing means 14, being configured to convey the light radiation emitted by the LED 6 towards the outside of the device 1 in a different way with respect to the first conveying means 13.

In an advantageous embodiment, in which, during step b), the first fixing screws 141 are removed by unscrewing them from the first support structure 16 and the support element 19 is subsequently removed by extracting it from the first support structure 16, thereby also removing the connection structure 20, during step c) the lens 23 (or the tubular structure comprising a series of lenses in series with each other) is inserted into the first support structure 16 and the screen 25 rests against the first support structure 16 (unless the latter is already fixed to the lens 23 or to the tubular structure comprising a series of lenses in series with each other). The screen 25 is then advantageously constrained to the first support structure 16 by means of the first fixing screws 141, which are screwed into the first support structure 16.

In a further advantageous embodiment, in which, during step b), by acting on the removable fixing means 14, the first support structure 16 and the first conveying means 13 supported thereby are separated from the support base 2, during step c), by acting on the removable fixing means 14, for example by tightening the second screws 142, it is possible to fix the second support structure 18, together with the second conveying means 15 supported thereby, to the support base 2.

The present finding also relates to a light radiation conveying kit.

The light radiation conveying kit according to the finding comprises a support base 2, advantageously analogous to that previously described, and an LED 6, advantageously analogous to that previously described, supported by the support base 2.

The light radiation conveying kit according to the finding comprises an electronic board 8, advantageously analogous to that previously described, supported by the support base 2 and configured to control the operation of the LED 6.

The light radiation conveying kit according to the finding comprises a first light radiation conveying group 12, advantageously analogous to that previously described, comprising first light radiation conveying means 13, advantageously analogous to those previously described, removably associable with the support base 2 by means of removable fixing means 14, advantageously analogous to those previously described, in such a way as to be positionable facing the LED 6 and to convey the light radiation emitted thereby towards the outside of the device 1.

The first conveying means 13 are removable from the support base 2 by acting on the removable fixing means 14.

The light radiation conveying kit according to the finding comprises a second light radiation conveying group 17, advantageously analogous to that previously described, comprising second light radiation conveying means 15, advantageously analogous to those previously described, removably associable with the support base 2 by means of the removable fixing means 14, in such a way as to be positionable facing the LED 6 and to convey the light radiation emitted thereby towards the outside of the device 1 in a different way with respect to the first conveying means 18.

It has thus been demonstrated that the finding achieves the objects and task set forth above.

Indeed, due to the possibility of using a single device, replacing only some components thereof, and in particular the light radiation conveying means, in order to perform light stimulations in the field of optogenetics, alternatively in vivo or in vitro, it is possible to reduce the overall storage space required for the equipment necessary for such stimulations with respect to the cited prior art.

This also allows to reduce the overall costs required to equip oneself for carrying out light stimulations in the field of optogenetics both in vivo and in vitro.

Furthermore, the finding has numerous additional advantages over existing solutions.

In particular, the removable and replaceable structure of the first and second light radiation conveying means enables a reduction in the time and costs associated with maintenance or replacement of possibly damaged parts thereof.

Furthermore, the removable and replaceable structure of the first and second light radiation conveying means reduces the overall environmental impact of the device, since, in the event of failure or breakage of the first or second conveying means, it is possible to replace only such conveying means and not the entire device.

Moreover, the removable and replaceable structure of the first and second light radiation conveying means simplifies cleaning and sanitization operations.

Furthermore, the removable structure of the light radiation conveying means allows a greater flexibility in the use of the device according to the finding, since it is possible to employ conveying means adapted to specific applications without the need to replace the entire device for light stimulation.

Finally, it is clear that the device for light stimulation, the method of reconfiguring such device, and the kit according to the finding are susceptible to numerous modifications and variants, all falling within the finding; furthermore, all details are replaceable by technically equivalent elements without departing from the scope of the attached claims.

## Claims

1. Device (1) for light stimulation, particularly for use in optogenetics, comprising:
- a support base (2);
- an LED (6), supported by said support base (2);
- an electronic board (8), supported by said support base (2) and configured to control the operation of said LED (6);
- a first light radiation conveying group (12) comprising first light radiation conveying means (13) removably associated with said support base (2), by means of removable fixing means (14), in such a way as to face said LED (6) and convey the light radiation emitted by the same towards the outside of said device (1),
wherein said first conveying means (13) are removable from said support base (2) by acting on said removable fixing means (14), in such a way as to allow the replacement of said first conveying means (13) with second light radiation conveying means (15) configured to convey the light radiation emitted by said LED (6) towards the outside of said device (1) in a different way with respect to said first conveying means (13).

2. Device (1), as in claim 1, wherein said first conveying group (12) comprises a first support structure (16), fixed to said support base (2), from which said first conveying means (13) are supported.

3. Device (1), as in claim 2, wherein said first support structure (16) is fixed to said support base (2) and wherein said first conveying means (13) and said second conveying means (15) can be fixed, alternatively to each other, in a removable manner, by the action of said removable fixing means (14), to said support structure (12).

4. Device (1), as in a previous claim, wherein said first conveying means (13) of light radiation comprise:
- a support element (19), removably associated with said support base (2) by the action of said removable fixing means (14),
- a connection structure (20), supported by said support element (19), configured to stably support a first free end (21a) of a first optical fibre (21) in such a way that said first free end (21a) faces or can face said LED (6) and configured to accommodate and fix, in a removably way, a second end of a second optical fibre in such a way that said second free end faces a second free end (21b) of said first optical fibre (21).

5. Device (1), as in claim 4, wherein said first conveying means (13) are configured to allow said first free end (21a) of said first optical fibre (21) to be translated along a first direction (Z) perpendicular to the plane of arrangement of said LED (6), so as to be able to bring it closer to, or further away from, said LED (6).

6. Device (1), as in claim 4 or 5, wherein said first conveying means (13) are configured to allow said first free end (21a) of said first optical fibre (21) to be moved along a first plane (XY) parallel to the plane of arrangement of said LED (6).

7. Device (1), as in a preceding claim, wherein said second conveying means (15) of light radiation comprise:
- a lens (23), or a tubular structure comprising a series of lenses in series with each other, configured to be able to be positioned with its first face (24) facing said LED (6) and to convey the light radiation emitted by said LED (6) along a given direction;
- a screen (25), transparent or partially transparent, facing a second face (26) of said lens (23) or tubular structure placed on the opposite side to said first face (24).

8. Method of reconfiguring a device (1) as in one or more of the preceding claims comprising the following steps:
a) arranging a device (1) as in one or more of the preceding claims, comprising a said first conveying group (12) of the light radiation;
b) removing said first conveying means (13) from said support base (2) of said device (1) by acting on said removable fixing means (14);
c) fixing to said support base (2) of said device (1), by acting on said removable fixing means (14), said second conveying means (15) of the light radiation configured to convey the light radiation emitted by said LED towards the outside of said device (1) in a different way with respect to said first conveying means (18).

9. Method of reconfiguring, as in claim 8, a device (1) as in claim 3 or a claim dependent on claim 3,
wherein said step c) comprises the step of removing said first conveying means (13) from said first support structure (16) by acting on said removable fixing means (14)
and wherein said step d) comprises the step of fixing said second conveying means (15) to said first support structure (16) by acting on said removable fixing means (14).

10. Light radiation conveying kit, comprising:
- a support base (2);
- an LED (6), supported by said support base (2);
- an electronic board (8), supported by said support base (2) and configured to control the operation of said LED (6);
- a first light radiation conveying group (12) comprising first light radiation conveying means (13) that can be removably associated with said support base (2), by means of removable fixing means (14), so that they can be positioned facing said LED (6) and convey the light radiation emitted by the same towards the outside of said device (1), wherein said first conveying means (13) are removable from said support base (2) by acting on said removable fixing means (14);
- a second light radiation conveying group (17) comprising second light radiation conveying means (15) which can be removably associated with said support base (2), by means of said removable fixing means (14), in such a way as to be positioned facing said LED (6) and convey the light radiation emitted by the same towards the outside of said device (1), in a different way with respect to said first conveying means (13).
